(19) Europäisches Patentamt
European Patent Office
Office européen des brevets

(11) Veröffentlichungsnummer: **0 308 808 B1**

# (12) EUROPÄISCHE PATENTSCHRIFT

(45) Veröffentlichungstag der Patentschrift: **18.12.91**

(51) Int. Cl.5: **C07C 43/29**, C07C 217/14, C07C 41/18

(21) Anmeldenummer: **88115089.0**

(22) Anmeldetag: **15.09.88**

## (54) Verfahren zur Herstellung von 2-Fluoro-5-allyl-diphenylethern.

(30) Priorität: **19.09.87 DE 3731608**

(43) Veröffentlichungstag der Anmeldung:
**29.03.89 Patentblatt 89/13**

(45) Bekanntmachung des Hinweises auf die Patenterteilung:
**18.12.91 Patentblatt 91/51**

(84) Benannte Vertragsstaaten:
**BE CH DE ES FR GB IT LI NL**

(56) Entgegenhaltungen:
**EP-A- 0 224 024**
**FR-A- 2 310 092**
**US-A- 2 450 497**
**US-A- 3 637 866**

**JOURNAL OF THE AMERICAN CHEMICAL SOCIETY, Band 79, Nr. 4, 27. Februar 1957, Seiten 960-964, American Chemical Society, US; J.T. YANG et al.: "Physical and inorganic chemistry"**

(73) Patentinhaber: **HOECHST AKTIENGESELLSCHAFT**
**Postfach 80 03 20**
**W-6230 Frankfurt am Main 80(DE)**

(72) Erfinder: **Schubert, Hans Herbert, Dr.**
**Geisenheimer Strasse 95**
**W-6000 Frankfurt am Main 71(DE)**

EP 0 308 808 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein neues Verfahren zur Herstellung von 2-Fluoro-5-allyl-diphenylethern der allgemeinen Formel I

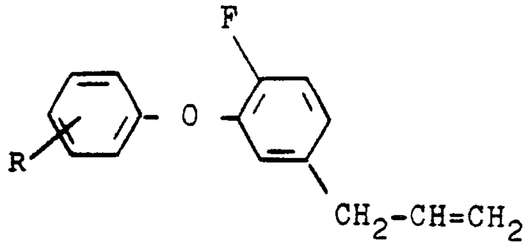

(I),

worin
R = H, Halogen, ($C_1$-$C_4$)-Alkyl oder ($C_1$-$C_4$)-Alkoxy bedeutet.
Die Verbindungen der Formel I sind wertvolle Zwischenprodukte bei der Synthese von biologisch aktiven Silanverbindungen (vgl. EP-A-02 24 024).

Die bisher bekannte Synthese dieser Verbindungen beruht auf einer Kupplung einer metallorganischen Verbindung der Formel II

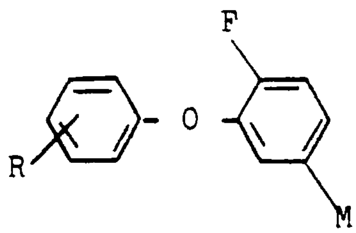

(II),

worin M einem Alkali- oder Erdalkalimetalläquivalent entspricht und R die obengenannte Bedeutung hat, mit einem Allylhalogenid. Dieser Reaktionsweg gestaltet sich aufgrund der schwierigen Zugänglichkeit (vielstufige Synthese) und der komplizierten Handhabung (luft- und feuchtigkeitsempfindlich, selbstentzündlich) der Verbindungen der Formel II problematisch im Sinne einer technischen Realisierung.
Es wurde nun ein Verfahren gefunden, mit dem sich die Verbindungen der Formel I in wenigen Schritten bei guten Ausbeuten aus käuflichen oder aber leicht zugänglichen, literaturbekannten Vorstufen herstellen lassen.

Gegenstand der vorliegenden Erfindung ist daher ein Verfahren zur Herstellung von Verbindungen der obengenannten Formel (I), dadurch gekennzeichnet, daß man eine Verbindung der Formel (III)

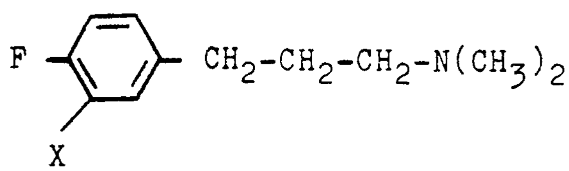

(III),

worin
X = Halogen bedeutet,
in Gegenwart eines Kupferkatalysators mit einem Alkali- oder Erdalkaliphenolat der Formel IV

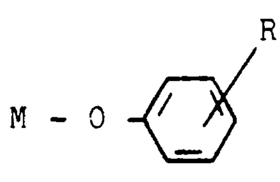

(IV),

worin M = Alkali- oder Erdalkalimetall ist und R die Bedeutung wie in Formel I besitzt, zu einer Verbindung der Formel V

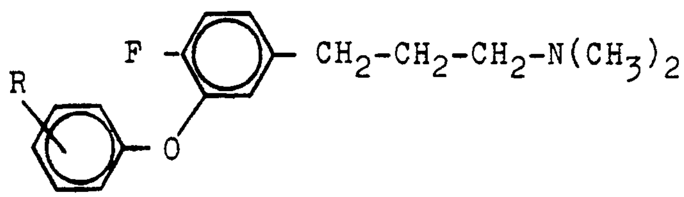

(V),

worin R die Bedeutung wie in Formel (I) besitzt, umsetzt und die nach Oxidation daraus entstandenen Aminoxide pyrolysiert.

Die Ausgangsverbindungen der Formel III lassen sich aus käuflichen Edukten nach literaturbekannten Verfahren herstellen. Auch die Oxidation der Verbindungen der Formel V zu den entsprechenden Aminoxiden und deren Pyrolyse sind allgemein bekannte Verfahren der organischen Chemie (A. Citterio, Org. Synth. 62, 67 (1984); E. Möller, R. Schröter, Methoden der org. Chemie, Houben-Weyl, Bd. XI/1, 648-664, (1957); A.C. Cope, C.L. Bumgardner, J. Amer. Chem. Soc. 79, 960 (1957)).

So lassen sich die Verbindungen der Formel III beispielsweise ausgehend von 3-Chlor-4-fluoranilin oder dem leicht aus 4-Fluoranilin durch Halogenierung in Gegenwart eines "Swamp-Katalysators" ($AlCl_3$/HClgas) erhältlichen 3-Brom-4-fluoranilin bzw. 3-Jod-4-fluoranilin synthetisieren. Bevorzugt wird hierbei die Brom- oder Chlorverbindung, besonders bevorzugt die Bromverbindung verwendet. Die aus diesen Edukten erhältlichen Diazoniumsalze lassen sich in guten Ausbeuten unter reduzierenden Bedingungen mit Acrolein zu 3-Phenylpropanalen der Formel VI alkylieren

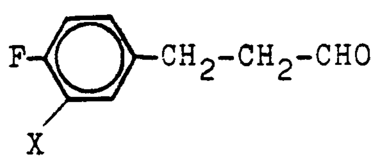

(VI),

worin X = Halogen bedeutet.

Deren reduktive Aminierung (z.B Umsetzung mit Dimethylamin und Wasserstoff in Gegenwart eines Katalysators oder mit Dimethylformamid und Ameisensäure) liefert die als Ausgangsstoffe dienenden 3-Arylpropyl-dimethylamine der Formel III.

Ausgehend von den Verbindungen der Formel III war beim erfindungsgemäßen Verfahren insbesondere die selektive und mit guten Ausbeuten verlaufende Bildung der Verbindungen der Formel V überraschend. Im Gegensatz zu anderen, literaturbekannten Umsetzungen von Fluorhalogenaromaten mit Phenolaten, bei denen bevorzugt das Fluor und nicht das andere Halogen ausgetauscht wird (vgl. DE-OS 2 619 489, US-PS 3 637 866), läßt sich hier insbesondere im Falle der Bromfluor- und Jodfluoraromaten der Formel III glatt das wenigen elektronegative Halogen substituieren. Die besten Ausbeuten werden dabei erzeilt, wenn man die Umsetzung in schwach polaren, aprotischen Solventien wie z.B. Polyethylenglykoldialkylethern bei Temperaturen zwischen 100° C und 255° C durchführt, wobei der optimale Temperaturbereich und das Lösungsmittel mit dem zu ersetzenden Halogen variieren.

Während der Jodfluoraromat der Formel III bereits bei Temperaturen von 100 - 160° C, bevorzugt bei 130 - 160° C, in Diethylenglykoldimethylether zur Reaktion gebracht werden kann, sind zur Umsetzung des Chlorfluoraromaten höher siedende Lösungsmittel wie z.B. Triglyme oder Tetraglyme und Temperaturen von 190-255° C, bevorzugt 220-250° C, notwendig. Der Bromfluoraromat reagiert z.B. in Diglyme oder Triglyme, aber auch in Diethylenglykoldiethylether bei Temperaturen von 145-210° C, bevorzugt bei 145-190° C, mit Phenolaten.

Die Umsetzung der Verbindungen der Formel III mit denen der Formel IV erfolgt in Gegenwart eines Katalysators, der Kupfer in unterschiedlichen Oxidationsstufen enthält. Bevorzugt sind die Oxidationsstufen $Cu^0$ und $Cu^{1+}$. Beispiele für einen solchen Katalysator sind Kupferpulver, Kupfer(I)oxid, Kupfer(I)chlorid und Kupfer(I)bromid, von denen Kupfer(I)oxid und Kupfer(I)chlorid bevorzugt verwendet werden.

Pro Mol einen Halogenfluoraromaten der Formel III werden 1-1,6 Mol, bevorzugt 1-1,4 Mol, eines Alkali- oder Erdalkaliphenolates, bevorzugt eines Natrium- oder Kaliumphenolates, eingesetzt. Die notwendige Menge des Kupferkatalysators schwankt allgemein zwischen 1-20 g. Die Aufarbeitung des Reaktionsproduktes (Verbindung der Formel V) geschieht auf übliche Weise z.B. durch Filtration, Verdünnen des Filtrates mit einem mit Wasser nicht mischbaren Lösungsmittel und Waschen der Lösung mit verdünnter Natronlauge und Wasser. Nach Trocknung und Filtration wird das Lösungsmittel abgedampft und der Rückstand unter Vakuum destilliert.

Die Diphenylether der Formel V werden anschließend bei Temperaturen unterhalb 100° C z.B. mit $H_2O_2$ oder Persäuren zu den entsprechenden Aminoxiden oxidiert, aus denen durch Pyrolyse die Verbindungen der Formel I gebildet werden.

Die Diphenylether der Formel V sind neu und ebenfalls Gegenstand der vorliegenden Erfindung.

Das erfindungsgemäße Verfahren wird durch die folgenden Ausführungsbeispiele verdeutlicht:

**Ausführungsbeispiele**

A) Herstellung einer Verbindung der Formel III

a) Eine Mischung aus 190 g (1.00 Mol) 3-Brom-4-fluoranilin, 300 ml konz. Salzsäure und 110 ml Wasser wird bei 0-10° C durch Zutropfen einer Lösung von 72 g (1.05 Mol) Natriumnitrit in 160 ml Wasser diazotiert. Die so erhaltene Diazoniumsalzlösung tropft man bei 0-20° C zu einer Mischung aus 2 1 käuflicher 15%iger $TiCl_3$-Lösung, 1,5 l DMF und 112 g (2.00 Mol) Acrolein.

Nach Abschluß der Gasentwicklung wird die Mischung mit 1.5 l Ether versetzt und die org. Phase abgetrennt. Die wäßrige Schicht wird noch dreimal mit Ether extrahiert. Dann wäscht man die vereinigten Auszüge zweimal mit 3%iger Sodalösung und einmal mit Wasser. Nach Trocknen und Eindampfen wird der Rückstand im Vakuum destilliert. Man erhält 107 g (46 %) eines blaßgelben Öls vom $Kp_{0,3}$ = 119-122° C, dem aufgrund seiner spektroskopischen Daten die Struktur des 3-(3-Brom-4-fluorphenyl)-propanals zugeordnet werden konnte.

b) Zu einer ca. 140° C heißen Mischung aus 93,6 g (1,28 Mol) Dimethylformamid und 33,9 g Ameisensäure tropft man eine Lösung von 106.5 g (0.46 Mol) 3-(3-Brom-4-fluorphenyl)-propanal in 102,8 g Ameisensäure. Dann erhitzt man bis zum Abschuß der Gasentwicklung unter Rückfluß (ca. 3 h), destilliert die überschüssigen Reagenzien bis zu einer Innentemperatur von 185° C ab und läßt die Mischung auf Raumtemperatur abkühlen.

Nach Zugabe von 300 ml 2N-Salzsäure extrahiert man zweimal mit Toluol und stellt die wäßrige Phase durch Zugabe von ca. 400 ml 2N-Natronlauge basisch. Das Produkt kann nun mit Ether extrahiert werden. Durch Waschen der Etherextrakte mit Wasser und Kochsalzlösung, Trocknen und Eindampfen erhält man das gewünschte Amin, das abschließend im Vakuum destilliert wird. 3-(3-Brom-4-fluorphenyl)-propyl-dimethylamin siedet bei 82-85° C/0,1 mm. Ausbeute: 90,5 g (76 %)

**B) Herstellung einer Verbindung der Formel I**

1) Eine Mischung aus 96,2 g (0,37 Mol) 3-(3-Brom-4-fluorphenyl)propyl-dimethylamin (Formel III), 64,4 g (0,56 Mol) Natriumphenolat, 75 g Diethylenglykoldiethylether und 1 g Kupfer(I)oxid wird 24 h bei 190° C gerührt. Nach der üblichen, bereits im allegemeinen Teil skizzierten Aufarbeitung erhält man 58,3 g (58 %) 3-(4-fluoro-3-phenoxyphenyl)propyl-dimethylamin (Formel V) als farbloses Öl vom $Kp_{0,07}$ = 119-132° C. Ein Gaschromatogramm der Verbindung ergibt eine Reinheit von 97,3 %.

2) Zu 57,4 g (0,21 Mol) 3-(4-fluoro-3-phenoxyphenyl)propyl-dimethylamin tropft man unter Eiswasserkühlung 81,6 g (0,84 Mol) 35%ige Wasserstoffperoxidlösung. Anschließend rührt man 24 h bei Raumtemperatur, setzt eine Spatelspitze Platin auf Aktivkohle (5 %) hinzu und rührt weitere 24 h bei Raumtemperatur. Nach Verdünnen der Mischung mit 200 ml Methanol wird filtriert und eingedampft. Der Rückstand wird sodann im Ölpumpenvakuum langsam auf 130-180° C erhitzt und das übergehende Produkt in einer gekühlten Vorlage aufgefangen. Zum Abtrennen des Hydroxylamins nimmt man das Destillat in 300 ml Heptan auf und wäscht zweimal mit je 100 ml 2N-Salzäure und zweimal mit Wasser. Nach Trocknen, Filtrieren und Einengen wird das Produkt im Vakuum destilliert. Man erhält 37,8 g (79 %) 2-Fluoro-5-allyl-diphenylether als farbloses Öl vom $Kp_{0,1}$ = 91° C.

**Patentansprüche**

1. Verfahren zur Herstellung von Verbindungen der Formel I

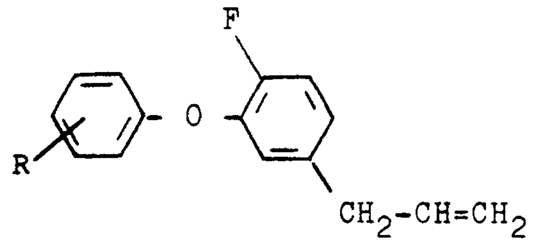

(I),

worin
R = H, Halogen, ($C_1$-$C_4$)-Alkyl oder ($C_1$-$C_4$)-Alkoxy bedeutet, dadurch gekennzeichnet, daß man eine Verbindung der Formel III

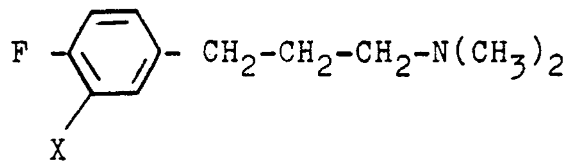

(III),

worin
X = Halogen bedeutet,
in Gegenwart eines Kupferkatalysators mit einem Alkali- oder Erdalkaliphenolat der Formel IV

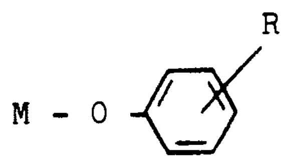

(IV),

worin M = Alkali- oder Erdalkalimetall ist und R die Bedeutung wie in Formel I besitzt,
zu einer Verbindung der Formel V

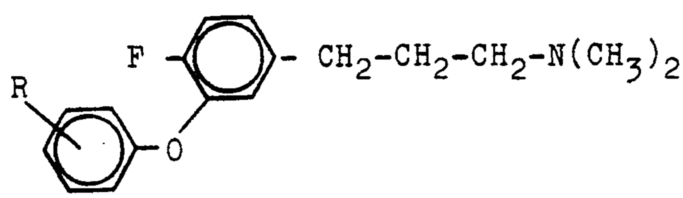

(V),

worin R die Bedeutung wie in Formel (I) besitzt, umsetzt und die nach Oxidation daraus entstandenen Aminoxide pyrolysiert.

2. Verfahren gemäß Anspruch 1, dadurch gekennzeichnet, daß man die Verbindungen der Formel III mit der Verbindung der Formel IV zwischen 100 °C und 255 °C umsetzt.

3. Verfahren gemäß Anspruch 1 oder 2, dadurch gekennzeichnet, daß man die Verbindung der Formel III mit der Verbindung der Formel IV zwischen 130 °C und 190 °C umsetzt.

4. Verfahren gemäß einem oder mehreren der Ansprüche 1 bis 3, dadurch gekennzeichnet, daß man für die Umsetzung der Verbindung der Formel III mit der Verbindung der Formel IV Polyethylenglykoldialkylether als Lösungsmittel verwendet.

5. Verfahren gemäß einem oder mehreren der Ansprüche 1 bis 4, dadurch gekennzeichnet, daß als Katalysator CuCl oder $Cu_2O$ verwendet wird.

6. Verbindungen der Formel V

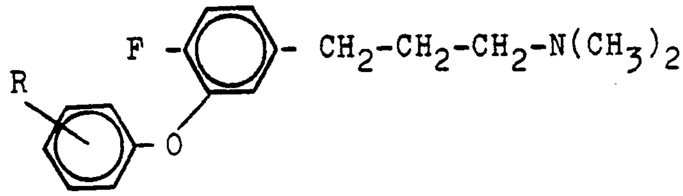

(V) ,

worin R = H, Halogen, ($C_1$-$C_4$)-Alkyl oder ($C_1$-$C_4$)-Alkoxy bedeutet.

7. Verbindungen der Formel V von Anspruch 6, dadurch gekennzeichnet, daß R = H, Fluor, Cl, $CH_3$ oder $OCH_3$ bedeutet.

8. Verwendung der Verbindungen der Formel V von Anspruch 6 zur Herstellung von Pflanzenschutzmittel-Wirkstoffen.

## Claims

1. A process for the preparation of compounds of the formula I

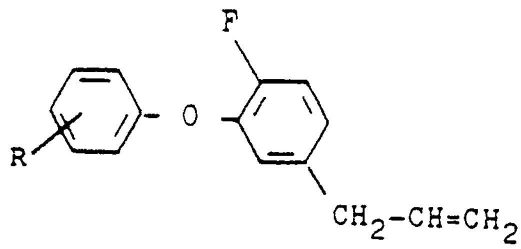

(I)

in which
R denotes H, halogen, ($C_1$-$C_4$)-alkyl or ($C_1$-$C_4$)-alkoxy, which comprises reacting a compound of the formula III

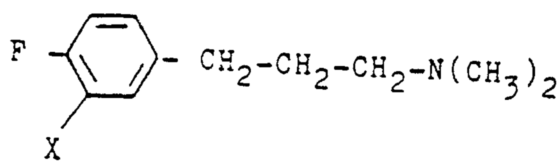

(III)

in which
X denotes halogen, in the presence of a copper catalyst with an alkali metal phenolate or alkaline earth metal phenolate of the formula IV

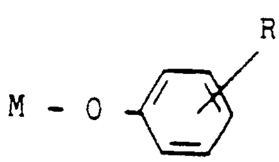

(IV)

in which M is an alkali metal or an alkaline earth metal and R has the meaning as in formula I, to give a compound of the formula V

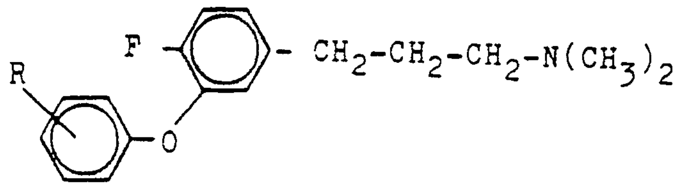

(V)

in which R has the meaning as in formula (I), and pyrolyzing the amine oxides formed from these products after oxidation.

2. The process as claimed in claim 1, wherein the compound of the formula III is reacted with the compound of the formula IV between 100 ° C and 255 ° C.

3. The process as claimed in claim 1 or 2, wherein the compound of the formula III is reacted with the compound of the formula IV between 130 ° C and 190 ° C.

4. The process as claimed in one or more of claims 1 to 3, wherein a polyethylene glycol dialkyl ether is used as the solvent for the reaction of the compound of the formula III with the compound of the formula IV.

5. The process as claimed in one or more of claims 1 to 4, wherein the catalyst used is CuCl or $Cu_2O$.

6. A compound of the formula V

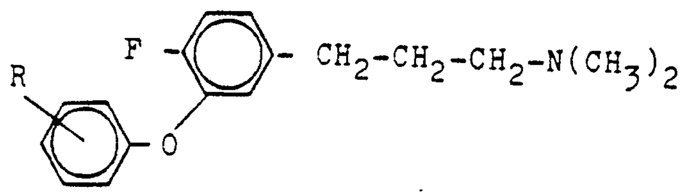

(V)

in which R denotes H, halogen, ($C_1$-$C_4$)-alkyl or ($C_1$-$C_4$)-alkoxy.

7. A compound of the formula V of claim 6, wherein R denotes H, fluorine, Cl, $CH_3$ or $OCH_3$.

8. Use of the compounds of the formula V of claim 6 for the preparation of active compounds for plant protection.

## Revendications

1. Procédé de préparation de composés de formule I ci-dessous :

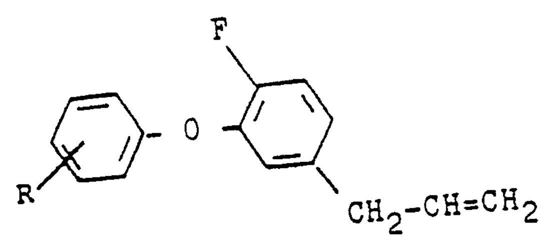

(I),

dans laquelle le symbole R représente l'hydrogène ou un halogène ou un alkyle ou un alcoxy en $C_1$-$C_4$, procédé caractérisé en ce que l'on fait réagir un composé de formule III :

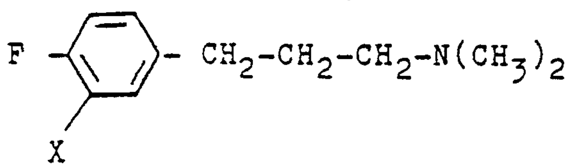

(III),

X désignant un atome d'halogène,
en présence d'un catalyseur au cuivre, avec un phénolate de métal alcalin ou alcalino-terreux de formule IV :

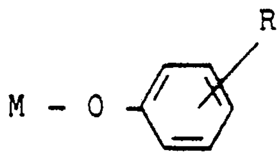

(IV),

M désignant un métal alcalin ou alcalino-terreux et R ayant la signification donnée pour la formule I, pour former un composé de formule V :

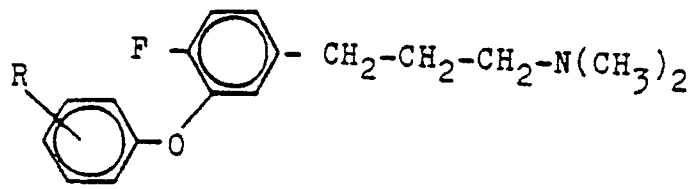

(V),

composé que l'on soumet à une oxydation, puis on pyrolyse l'amino-oxyde ainsi formé.

2. Procédé selon la revendication 1, caractérisé en ce que l'on fait réagir le composé de formule III avec le composé de formule IV entre 100 à 255° C.

3. Procédé selon la revendication 1 ou 2, caractérisé en ce que l'on fait réagir le composé de formule III avec celui de formule IV entre 130 et 190° C.

4. Procédé selon une ou plusieurs des revendications 1 à 3, caractérisé en ce que l'on utilise comme solvant un éther dialkylique de polyéthylène-glycol pour faire réagir le composé de formule III avec celui de formule IV.

5. Procédé selon une ou plusieurs des revendications 1 à 4, caractérisé en ce que l'on utilise comme catalyseur du chlorure cuivreux CuCl ou de l'oxyde cuivreux $Cu_2O$.

6. Les composés de formule V :

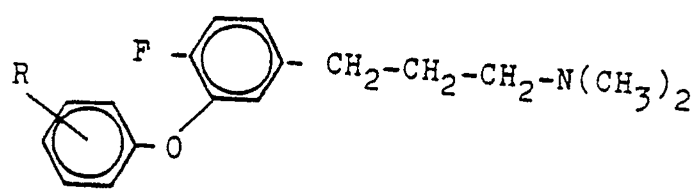

(V) ,

dans laquelle R désigne l'hydrogène ou un halogène ou un alkyle ou un alcoxy en $C_1$-$C_4$.

7. Composés de formule V selon la revendication 6, caractérisés en ce que R est un atome d'hydrogène, de fluor ou de chlore ou le groupe $CH_3$ ou $OCH_3$.

8. L'emploi des composés de formule V selon la revendication 6 pour fabriquer des matières actives de produits destinés à protéger des végétaux.